(19) Europäisches Patentamt
European Patent Office
Office européen des brevets

(11) Veröffentlichungsnummer: **0 142 041**
**B1**

(12) # EUROPÄISCHE PATENTSCHRIFT

(45) Veröffentlichungstag der Patentschrift: **05.10.88**

(51) Int. Cl.⁴: **C 07 B 37/02** // C07C19/08, C07C21/18, C07C31/34, C07C33/42, C07C69/63

(21) Anmeldenummer: **84112301.1**

(22) Anmeldetag: **12.10.84**

(54) **Verfahren zur Herstellung von fluoralkylsubstituierten Alkanen oder Alkenen.**

(30) Priorität: **21.10.83 DE 3338299**

(43) Veröffentlichungstag der Anmeldung: **22.05.85 Patentblatt 85/21**

(45) Bekanntmachung des Hinweises auf die Patenterteilung: **05.10.88 Patentblatt 88/40**

(84) Benannte Vertragsstaaten: **BE CH DE FR GB IT LI NL**

(56) Entgegenhaltungen:
**EP - A - 0 115 943**
**DE - A - 2 132 036**

**Die Akte enthält technische Angaben, die nach dem Eingang der Anmeldung eingereicht wurden und die nicht in dieser Patentschrift enthalten sind.**

(73) Patentinhaber: **HOECHST AKTIENGESELLSCHAFT, Postfach 80 03 20, D-6230 Frankfurt am Main 80 (DE)**

(72) Erfinder: **von Werner, Konrad, Dr., Buch 1 1/2, D-8261 Halsbach (DE)**

**EP 0 142 041 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein Verfahren zur Herstellung von fluoralkylsubstituierten Alkanen oder Alkenen.

Es ist bekannt, Iodperfluoralkane an die C–C-Doppelbindung von Alkenen, die verschiedene Substituenten tragen können, anzulagern. Hierbei wurden verschiedene Wege beschritten. Die rein thermische Anlagerung benötigt hohe Temperaturen, führt oft nur zu mässigen Umsätzen und verläuft im allgemeinen wenig selektiv, dass heisst, es entsteht ein Gemisch verschiedener Anlagerungsprodukte.

Bekannt ist auch die photochemische Anlagerung von Perfluoralkyliodiden an Alkene und Alkine, die allerdings oft lange Reaktionszeiten erfordert, um zu brauchbaren Umsätzen zu kommen, die ebenfalls zu Gemischen verschiedener Anlagerungsprodukte führt und überdies technisch umständlich und energieaufwendig ist.

Weiterhin ist bekannt, Perfluoralkyliodide unter Verwendung von bekannten, radikalisch zerfallenden Katalysatoren wie Azobis-isobutyronitril oder organischen Peroxiden in aprotischen Lösungsmitteln an Alkene anzulagern. Die Reaktionstemperaturen sind gegenüber der thermischen Anlagerung erheblich herabgesetzt, es werden auch häufig gute Umsätze erhalten. Die Anlagerungsreaktion verläuft jedoch häufig nicht selektiv, was die Ausbeute an erwünschten Produkten schmälert.

Schliesslich ist es bekannt, Perfluoralkylkupfer(I) in aprotischem Lösungsmittel (Dimethylsulfoxid) mit Alkenen umzusetzen, wobei ebenfalls vergleichsweise niedrige Reaktionstemperaturen ausreichend sind, allerdings wiederum Gemische von verschiedenen Reaktionsprodukten entstehen, die die Reindarstellung der einzelnen Produkte erschweren und die Ausbeute an erwünschten Produkten mindern. Das Perfluoralkylkupfer(I) kann auch aus Perfluoralkyliodid und metallischem Kupfer, wiederum unter Verwendung eines aprotischen Lösungsmittels (Dimethylsulfoxid), erst in der Reaktionsmischung erzeugt werden, wobei auch weniger (beispielsweise nur ein Drittel) als die stöchiometrisch berechnete Menge des metallischen Kupfers ausreichen. Jedoch werden auch bei dieser Reaktionsart in nicht unbeträchtlicher Menge Nebenprodukte gebildet.

Es wurde nun ein Verfahren gefunden, welches ermöglicht, ein ganz oder überwiegend mit Fluor substituiertes Alkyliodid oder Alkylbromid an Alkene oder Alkine so anzulagern, dass in guten Ausbeuten im wesentlichen nur ein Anlagerungsprodukt entsteht, welches leicht gereinigt und zu technisch interessanten Produkten weiter umgesetzt werden kann. Weiterhin von Vorteil sind die insbesondere gegenüber dem thermischen Verfahren niedrigeren Reaktionstemperaturen.

Es handelt sich um ein Verfahren zur Herstellung von fluoralkylsubstituierten Alkanen oder Alkenen durch Reaktion einer Verbindung der Formel

$$XR_fZ,$$

worin bedeuten:

$R_f$ einen perfluorierten Alkylenrest, geradkettig mit 1 bis 15 C-Atomen, verzweigt mit 3 bis 15 C-Atomen oder cyclisch mit 4 bis 8 C-Atomen,

X Wasserstoff, Fluor, Chlor, Brom oder Iod,

Z Brom oder Iod,

mit einer Verbindung der Formeln

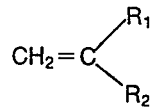
,

worin $R_1$ und $R_2$ gleich oder verschieden sein können und jedes für sich bedeutet:
Wasserstoff, Fluor, Chlor, einen Perfluoralkylrest mit 1 bis 12 C-Atomen, in dem ein Fluoratom durch ein Wasserstoff- oder Chloratom ersetzt sein kann, einen Alkylrest mit 1 bis 20 C-Atomen, einen Alkenylrest mit 2 bis 20 C-Atomen, einen Arylrest mit 6 bis 10 C-Atomen oder einen Arylalkylrest mit 7 bis 12 C-Atomen, wobei ein Substituent der vier zuletzt genannten Arten seinerseits substituiert sein kann mit F; Cl; –OH oder –OR′, worin R′ einen Alkyl- oder Alkylcarboxy-Rest mit 1 bis 5 C-Atomen bedeutet, oder

$$CH{\equiv}C{-}R_3,$$

worin $R_3$ bedeutet:
einen Perfluoralkylrest mit 1 bis 12 C-Atomen, in dem ein Fluoratom durch ein Wasserstoff- oder Chloratom ersetzt sein kann, einen Alkylrest mit 1 bis 20 C-Atomen, einen Alkenylrest mit 2 bis 20 C-Atomen, einen Arylrest mit 6 bis 10 C-Atomen oder einen Arylalkylrest mit 7 bis 12 C-Atomen, wobei ein Substituent der vier zuletzt genannten Arten seinerseits substituiert sein kann mit F; Cl; –OH oder –OR′, worin R′ einen Alkyl- oder Alkylcarboxy-Rest mit 1 bis 5 C-Atomen bedeutet, in der Wärme bei Normaldruck oder Überdruck in Gegenwart eines Metall-Komplex-Katalysators, das dadurch gekennzeichnet ist, dass bei 20 bis 130 °C gearbeitet wird und als Katalysator mindestens eine Metall-Komplexverbindung eingesetzt wird, die als Zentralatom ein Metall enthält, welches im Periodensystem der Elemente eine der Ordnungszahlen 24 bis 30; 42 bis 48 oder 74 bis 79 hat, und eine Wertigkeitsstufe von Null, +1 oder +2 aufweist, wobei diese Metall-Komplexverbindung mindestens einen der folgenden Liganden enthält:
Kohlenmonoxid; Trialkylphosphit; Triarylphosphit oder eine Verbindung der Formel

$$(R)_3Q,$$

worin bedeuten:

R = Alkyl-, Aryl- oder Arylalkyl-

Q = Phosphor, Arsen oder Antimon, wobei ferner solche Metall-Komplexverbindungen ausgeschlossen sind, die Kohlenmonoxid als Liganden und ein Metall, das im Periodensystem der Elemente eine der Ordnungszahlen 26 bis 28; 44 bis 46 und 76 bis 78 aufweist, als Zentralatom enthalten.

Unter «Metallkomplex-Verbindung» ist eine Koordinationsverbindung des jeweiligen Metalls zu verstehen, wie sie beispielsweise in «Advanced Inorganic Chemistry» (Cotton and Wilkinson) Interscience Publishers, 2. Auflage, 1966, Seiten 124 und 125 definiert ist.

Hat das als Zentralatom vorliegende Metall eine Wertigkeit über +2, wird ein Nachlassen der Wirksamkeit auf die Umsetzung des fluorierten Alkyliodids oder -bromids mit dem Alken oder Alkin festgestellt. Gute Ergebnisse werden erhalten, wenn das Metall-Zentralatom in der Wertigkeitsstufe +1 und insbesondere in der Wertigkeitsstufe 0 vorliegt.

Wegen ihrer guten Wirksamkeit werden bevorzugt Metallkomplex-Verbindungen eingesetzt, die Chrom, Molybdän, Rhenium oder Rhodium als Zentralatom enthalten. Besonders bevorzugt werden Ruthenium- oder Nickel-Komplexverbindungen eingesetzt.

Bevorzugt werden ferner die Metallkomplex-Verbindungen verwendet, die mindestens einen der folgenden Liganden enthalten: Trialkylphosphit; Triarylphosphit oder eine Verbindung der Formel $(R)_3Q$, worin bedeuten: R einen Alkyl-, Aryl- oder Arylalkyl-Rest und Q Phosphor, Arsen oder Antimon.

Sofern das Zentralatom der Metallkomplex-Verbindung kein Metall mit einer der Ordnungszahlen 24 bis 26; 44 bis 46 oder 76 bis 78 ist, kann einer oder mehrere der restlichen Liganden CO sein.

Neben dem weiter oben beschriebenen, in der Metall-Komplexverbindung mindestens vorhandenen Liganden können weitere Liganden der gleichen Art oder einer anderen oben beschriebenen Art oder einer anderen oben nicht beschriebenen Art in der Metall-Komplexverbindung zugegen sein. Weitere mögliche Liganden sind beispielsweise Diene wie Cyclopentadien, 1,4-Cyclooctadien, stickstoffhaltige Verbindungen wie Pyridin, o-Phenanthrolin. Sofern das Metall als Zentralatom eine höhere Oxidationsstufe als 0 aufweist, sind beispielsweise die entsprechenden Metallhalogenide, insbesondere die Chloride, gut geeignet. Da sie leicht zugänglich, gut zu handhaben und gut wirksam sind, werden bevorzugt Metall-Komplexverbindungen eingesetzt, die mindestens einen der folgenden Liganden enthalten: $(R''O)_3P$; $(R'')_3P$; $(R'')_3As$, worin R'' einen Alkylrest mit 1 bis 8 C-Atomen oder einen Arylrest mit 6 bis 10 C-Atomen oder einen Arylalkylrest mit 7 bis 12 C-Atomen bedeutet. Als Beispiele seien genannt: $[(C_2H_5O)_3P]_4Ni$; $[(C_6H_5)_3P]_4RuCl_2$; $[(C_6H_5)_3P]_3RhCl$; $[(C_6H_5)_3P]Cr(CO)_5$; $[(C_6H_5)_3P]Mo(CO)_5$; $[(C_2H_5O)_3P]Cr(CO)_5$;

Die Reaktion wird im Temperaturbereich von 20 bis 130 °C durchgeführt. Unterhalb 20 °C werden im allgemeinen zu geringe Ausbeuten erzielt, oberhalb 130 °C kann die Ausbeute an dem erwünschten Reaktionsprodukt im allgemeinen nicht mehr verbessert werden, im Gegenteil nimmt Zahl und Menge der gebildeten Nebenprodukte zu, so dass die Reindarstellung des erwünschten Produktes schwieriger und der Prozess unwirtschaftlich wird. Zweckmässig wird die Temperatur der Reaktionsmischung mindestens so hoch gehalten, dass die Reaktionspartner und die daraus entstehenden Produkte nicht in fester Form vorliegen. Wenn dies nicht möglich ist, werden zweckmässig inerte Lösungsmittel verwendet, die sich möglichst im Siedepunkt deutlich von den eingesetzten Reaktionspartnern und den zu erwartenden Reaktionsprodukten unterscheiden.

Als Beispiele seien niedere aliphatische Alkohole, Ether, fluorierte Ether oder fluorierte Kohlenwasserstoffe genannt. Vorzugsweise wird im Temperaturbereich von 50 bis 100 °C gearbeitet.

Das erfindungsgemässe Verfahren kann unter Druck ausgeführt werden, wenn mindestens einer der Reaktionspartner bei der gewählten Umsetzungstemperatur gasförmig ist. Zweckmässig wird unter dem autogenen Druck des oder der Reaktionspartner gearbeitet, es kann jedoch auch ein höherer Druck angewendet werden. Der Druckbereich, in dem das neue Verfahren ausgeführt wird, liegt zwischen 0,098 und 5 MPa. Nach oben ist der Druckbereich im wesentlichen nur durch wirtschaftliche Erwägungen begrenzt. Vorzugsweise wird im Druckbereich von 0,098 bis 2,5 MPa gearbeitet.

Die Dauer der Reaktion ist von der gewählten Temperatur und den Reaktionspartnern abhängig. Eine Reaktionsdauer von 1 bis 50 h ist in der Regel ausreichend. Unterhalb 1 h ist die Reaktion meist noch unvollständig, oberhalb 50 h wird im allgemeinen keine Ausbeuteverbesserung mehr beobachtet, es wächst die Gefahr der Bildung unerwünschter Nebenprodukte. Vorzugsweise wird eine Reaktionsdauer von 3 bis 30 h gewählt.

Das Molverhältnis der Reaktionspartner (z.B. fluoriertes Alkyliodid zu Alken) beträgt 10 : 1 bis 1 : 10. Im allgemeinen wird man eher einen molaren Überschuss des Alkens wählen, doch wächst dadurch die Gefahr, dass mehrere Alkenmoleküle mit einem fluorsubstituierten Alkyliodid reagieren, was zu weniger erwünschten Produkten führt. Es ist deshalb in einigen Fällen erforderlich, einen bisweilen beträchtlichen molaren Überschuss des fluorierten Alkyliodids zu wählen.

Vorzugsweise werden die Reaktionspartner im Molverhältnis 5 : 1 bis 1 : 5 eingesetzt.

Vom Katalysator werden 0,1 bis 10 Mol-%, bezogen auf jene der beiden reagierenden Verbindungen, die mit geringerer Molzahl eingesetzt wird, angewendet. Unterhalb 0,1 Mol-% ist der Katalysator in der Regel nicht mehr ausreichend wirksam, oberhalb 10 Mol-% wird keine zusätzliche Wirkung des Katalysators mehr festgestellt. Vorzugsweise werden 0,5 bis 3 Mol-%, bezogen auf eingesetztes fluoriertes Alkyliodid oder -bromid vom Katalysator verwendet.

Das erfindungsgemässe Verfahren kann, wie oben bereits erwähnt, auch in Gegenwart von Lösungsmitteln durchgeführt werden, jedoch wird vorteilhaft in Abwesenheit von aprotischen Lösungsmitteln gearbeitet, da diese häufig die Bildung unerwünschter Nebenprodukte begünstigen. Protische Lösungsmittel stören überraschenderweise den Reaktionsablauf nur wenig oder gar nicht.

Wegen der Sauerstoff-Empfindlichkeit vieler Metall-Komplexverbindungen wird das erfindungsgemässe Verfahren zweckmässig in einer Inertgasatmosphäre, beispielsweise unter Stickstoff oder Argon durchgeführt.

Es wird eine Verbindung folgender Formel eingesetzt:

$$XR_fZ,$$

worin bedeuten:

$R_f$ einen perfluorierten Alkylenrest, geradkettig mit 1 bis 15 C-Atomen oder verzweigt mit 3 bis 15 C-Atomen oder cyclisch mit 4 bis 8 C-Atomen;

X Wasserstoff, Fluor, Chlor, Brom oder Iod;

Z Brom oder Iod.

Die Verbindungen, in denen X = Fluor, Brom oder Iod ist, werden bevorzugt eingesetzt, da sie gut reagieren und zu technisch interessanten Reaktionsprodukten führen. Aus den gleichen Gründen und wegen der leichten Zugänglichkeit werden Verbindungen bevorzugt, in denen $R_f$ einen perfluorierten geradkettigen Alkylenrest mit 2 bis 12 C-Atomen bedeutet.

Das erfindungsgemässe Verfahren wird durchgeführt mit einem substituierten oder unsubstituierten Alken, das folgender Formel entspricht:

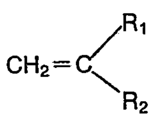 ,

worin $R_1$ und $R_2$ gleich oder verschieden sein können und jedes für sich bedeutet:
Wasserstoff, Fluor, Chlor, einen Perfluoralkylrest mit 1 bis 12 C-Atomen, in dem ein Fluoratom durch Wasserstoff oder Chlor ersetzt sein kann, einen Alkylrest mit 1 bis 20 C-Atomen, einen Alkenylrest mit 2 bis 20 C-Atomen, einen Arylrest mit 6 bis 10 C-Atomen oder einen Arylalkylrest mit 7 bis 12 C-Atomen. Ein Substituent der vier zuletzt genannten Arten (Alkyl, Alkenyl-, Aryl- oder Arylalkyl-) kann seinerseits substituiert sein mit Fluor, Chlor, –OH oder –OR′, worin R′ einen Alkyl- oder Alkylcarboxy-Rest mit 1 bis 5 C-Atomen bedeutet. Ferner können $R_1$ oder $R_2$ einen Silylrest bedeuten, der mit Alkylgruppen, die 1 bis 4 C-Atome enthalten, mit Alkoxygruppen die 1 bis 5 C-Atome enthalten oder mit Chlor substituiert ist.

Besonders bevorzugt werden solche Verbindungen eingesetzt, in denen $R_2$ Wasserstoff bedeutet und $R_1$ ebenfalls Wasserstoff oder einen Alkylrest mit 1 bis 10 C-Atomen oder einen Alkenylrest mit 2 bis 10 C-Atomen, wobei sowohl der Alkyl- wie auch der Alkenylrest mit –OH oder –OR′ substituiert sein kann, worin R′ einen Alkyl, oder Alkylcarboxy-Rest mit 1 bis 3 C-Atomen bedeutet. Ebenso bevorzugt sind Verbindungen, in denen $R_2$ Wasserstoff und $R_1$ einen Silylrest bedeuten, der mit Alkylgruppen, die 1 bis 4 C-Atomen enthalten, mit Alkoxygruppen, die 1 bis 5 C-Atome enthalten oder mit Chlor substituiert ist.

Das erfindungsgemässe Verfahren kann auch mit einem substituierten oder unsubstituierten Alkin durchgeführt werden, welches folgender Formel entspricht:

$$\equiv C{-}R_3$$

worin $R_3$ bedeutet: einen Perfluoralkylrest mit 1 bis 12 C-Atomen, in dem ein Fluoratom durch ein Wasserstoff- oder ein Chloratom ersetzt sein kann, einen Alkylrest mit 1 bis 20 C-Atomen, einen Alkenylrest mit 2 bis 20 C-Atomen, einen Arylrest mit 6 bis 10 C-Atomen oder einen Arylalkylrest mit 7 bis 12 C-Atomen, wobei ein Substituent der vier zuletzt genannten Arten seinerseits substituiert sein kann mit F; Cl; –OH oder –OR′, worin R′ einen Alkyl- oder Alkylcarboxy-Rest mit 1 bis 5 C-Atomen bedeutet.

Nach Beendigung der Reaktion wird die Mischung abgekühlt und, sofern sie flüssig bleibt und es sich als notwendig erweist, durch Abfiltrieren, Abzentrifugieren oder andere geeignete Methoden von festen Partikeln getrennt. Das Filtrat oder die nicht-filtrierte Reaktionsmischung wird nun, gegebenenfalls unter Anwendung von Unterdruck, fraktioniert destilliert. Sofern die Hauptfraktion kein ausreichend reines Produkt enthält, wird diese erneut fraktioniert destilliert. Wenn die Reaktionsmischung beim Abkühlen erstarrt, wird sie zweckmässig mit geeigneten, leicht-flüchtigen Lösungsmitteln extrahiert, entweder durch mehrfaches Digerieren oder beispielsweise in einem Soxhlet-Apparat. Geeignete Lösungsmittel sind beispielsweise Dichlormethan, Chloroform, Trichlorethylen, Tetrachlorkohlenstoff, niedrig siedende, jedoch bei Zimmertemperatur flüssige, fluorierte Kohlenwasserstoffe, die auch noch Chlormoleküle enthalten können, Ether wie Diethylether, Tetrahydrofuran, Glykoldimethylether. Aus den Extrakten wird nun zunächst das Lösungsmittel teilweise oder vollständig abgedampft und das Produkt durch Umkristallisieren gereinigt oder das Lösungsmittel vollständig abgedampft und das Produkt durch fraktionierte Destillation, wie oben beschrieben, in reiner Form gewonnen. Das im Destillations- oder Extraktions-Rückstand in einer Verbindung oder auch als solches vorhandene Metall kann, sofern es sich lohnt, wiedergewonnen und als Metall-Komplexverbindung erneut als Katalysator verwendet werden.

Mit dem erfindungsgemässen Verfahren werden mit guter Selektivität die einfachen Additionsverbindungen des fluorierten Alkyliodids oder Alkylbromids mit dem entsprechenden Alken oder Alkin erhalten, die folgenden Formeln entsprechen:

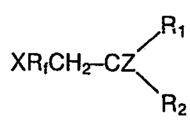 oder $XR_fCH=CZ{-}R_3$

worin $R_f$, $R_1$, $R_2$, $R_3$, Z und X die weiter oben genannte Bedeutung haben. Nur in untergeordnetem Masse können Reaktionsprodukte auftreten, bei denen das fluorierte Alkyliodid oder Alkylbromid mit zwei Alkenen oder zwei Alkinen reagiert hat,

wobei zum Beispiel folgende Verbindungen entstehen können:

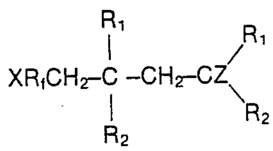 oder 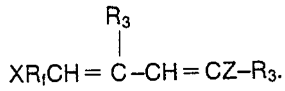

Wie weiter oben bereits ausgeführt, kann jedoch die Bildung von Verbindungen der zuletzt genannten Formeln durch Anwendung eines Überschusses des fluorierten Alkyliodids oder Alkylbromids zurückgehalten werden.

Die nach dem neuen Verfahren erzeugten Verbindungen sind wertvolle Zwischenprodukte, sie können beispielsweise verarbeitet werden, indem nach bekanntem Verfahren, wie zum Beispiel in der DE-OS 28 34 795 beschrieben, das Iodatom oder Bromatom im Molekül in eine Hydroxylgruppe übergeführt wird. Verbindungen mit einer C–C-Doppel- beziehungsweise Dreifachbindung werden erhalten durch Abspalten von Iodwasserstoff oder Bromwasserstoff mit Alkalien, wie beispielsweise Natrium oder Kaliumhydroxid, Natrium- oder Kaliumcarbonat, wobei man zweckmässig in wässrig-alkoholischer oder rein alkoholischer Lösung, beispielsweise in Methanol oder Ethanol, arbeitet, wobei die Reaktion im Fall von wasserhaltigen Medien durch Einsatz von Phasentransfer-Katalysatoren wie Tetraalkylammonium-Salze oder Tetraalkylphosphonium-Salze noch verbessert wird. Verbindungen mit Doppelbindungen jeweils am Molekülende sind als Comonomere für die Polymerisation von fluorhaltigen, ungesättigten Kohlenwasserstoffen, beispielsweise Tetrafluorethylen, geeignet. Andere aus den erfindungsgemäss hergestellten Verbindungen erhaltene Stoffe dienen zur Herstellung von Textilveredlungsmitteln, beispielsweise zur Oleophobierung oder Hydrophobierung, als Feuerlöschmittel oder als besonders beständige Emulgatoren, beispielsweise für die Elektrolyse.

Nachfolgende Beispiele sollen die Erfindung näher erläutern.

Vergleichsversuche A und B sowie Beispiel 5

Es wird ein heiz- und kühlbarer Schüttelautoklav aus $V_2A$-Edelstahl mit 250 $cm^3$ Inhalt verwendet. In diesen Autoklaven werden die in nachstehender Tabelle angegebenen Verbindungen der Formel $XR_fZ$ und der jeweils verwendete Katalysator in den aus der Tabelle ersichtlichen Mengen gegeben. Der Autoklav wird nun verschlossen, zunächst mit Stickstoff, dann mit Ethylen gespült und schliesslich ein Ethylendruck, wie in der Tabelle angegeben, im Autokolav eingestellt. Nun wird der Autoklav unter Schütteln aufgeheizt bis zu der in der Tabelle angegebenen Temperatur und während der ebenfalls dort verzeichneten Dauer auf dieser Temperatur gehalten, wobei auch der Ethylendruck konstant gehalten wird. Das durch die Reaktion verbrauchte Ethylen wird also ständig nachgeliefert, so dass im Autoklav stets ein molarer Überschuss an Ethylen im Verhältnis zur eingesetzten Verbindung $XR_fZ$ vorhanden ist. In einigen Fällen wird am Beginn der Reaktion ein Ansteigen der Temperatur der Reaktionsmischung über den in der Tabelle angegebenen Wert festgestellt. Dem wird durch Kühlung entgegengewirkt, bis der angegebene Wert wieder erreicht ist und dieser konstant gehalten.

Nach Beendigung der Reaktionsdauer wird der Autoklav abgekühlt, entspannt und geöffnet. Der Autoklaveninhalt wird nun, falls erforderlich, filtriert. Von einer Probe des Filtrats oder, wenn nicht filtriert wurde, vom Autoklaveninhalt wird ein $^{19}F$-Kernresonanzspektrum aufgenommen und anhand dieses Spektrums der prozentuale Umsatz, bezogen auf die eingesetzten $-CF_2I$-Gruppen, ermittelt. Eine weitere Probe des Filtrats bzw. des Autoklaveninhalts wird gaschromatographisch untersucht. Die gefundenen Flächenprozente im Gaschromatogramm, die angenähert den tatsächlich gebildeten Molprozenten entsprechen, sind in nachfolgender Tabelle angegeben. Hierbei bedeuten:

$m_1$ = Reaktionsprodukt von einem Molekül $XR_fZ$ mit einem Molekül der Formeln

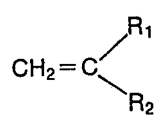 oder $CH \equiv C-R_3$,

wobei eine Verbindung folgenden Typs entsteht:

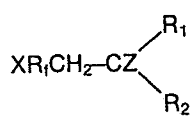 oder $XR_fCH = CZ-R_3$

$m_2$ = Reaktionsprodukt von einem Molekül $XR_fZ$ mit zwei Molekülen einer Verbindung der Formeln

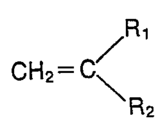 oder $CH \equiv C-R_3$,

wobei im allgemeinen eine Verbindung folgender Formeln entsteht:

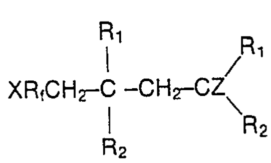 oder 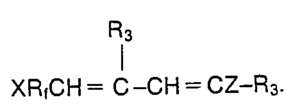

Tabelle

| Vergleichs-versuch-Beispiel-Nr. | $XR_fZ$ | mol | $CH_2=C\begin{smallmatrix}R_1\\R_2\end{smallmatrix}$ oder $CH\equiv C-R_3$ | mol | Kataly-sator | Mol-%[1+] | Temp. °C | Druck MPa | Dauer h | Umsatz[2+] % | Produkteigenschaften (Flächen-%)[2+] $m_1$ | $m_2$ | $XR_fZ$ | $XR_fH$ |
|---|---|---|---|---|---|---|---|---|---|---|---|---|---|---|
| A | I | 0,2 | a | > 0,2 | – | – | 190 | 2 | 11 | 91 | 83,8 | 1,2 | 9,0 | 5,5 |
| 1 | II | 0,1 | a | > 0,1 | K2 | 1 | 75 | 0,1 | 7 | 92,8 | 93,9 | 1,0 | 4,3 | < 0,8 |
| 2 | II | 0,1 | a | > 0,1 | K3 | 2 | 85 | 0,1 | 8 | 42,6 | 45,7 | – | 53,7 | < 0,6 |
| 3 | II | 0,1 | a | > 0,1 | K4 | 2 | 85 | 0,1 | 5 | 86,9 | 88,1 | – | 11,2 | < 0,7 |
| 4 | II | 0,1 | a | > 0,1 | K5 | 2 | 85 | 0,1 | 5 | 71,4 | 69,0 | – | 30,1 | < 0,9 |
| 5 | II | 0,2 | a | > 0,2 | K7 | 2 | 100 | 2 | 15 | 100 | 98,8 | 0,2 | 0,3 | < 0,7 |
| B | VII | 0,2 | a | 0,2 | AIBN | 5 | 80 | 2,0 | 15 | 60,5 | 12,6 | 43,9 | 37,2 | n.b. |

1+ bezogen auf jene der beiden miteinander reagierenden Verbindungen, die mit geringerer Molzahl eingesetzt wird
2+ bezogen auf die in der Verbindung $XR_fZ$ vorhandenen $-CF_2Z$-Gruppen (Z = I oder Br)

$XR_fH$ bedeutet eine Verbindung der Formel $XR_fZ$, in der das Brom- oder Iodatom Z am Perfluoralkylenrest $R_f$ durch Wasserstoff ersetzt ist.

In den obengenannten Formeln haben die Substituenten $R_f$; $R_1$; $R_2$; $R_3$; X und Z die weiter oben angegebene Bedeutung.

Beispiele 1 bis 4

Es wird ein temperierbarer Glaskolben von 250 $cm^3$ Inhalt verwendet, der mit Rührer, Rückflusskühler, Thermometer und einem Gaseinleitungsrohr versehen ist. In den Glaskolben werden Iodperfluoroctan und der aus nachstehender Tabelle ersichtliche Katalysator in den dort verzeichneten Mengen gegeben. Nun wird die in der Tabelle angegebene gasförmige Verbindung der Formel

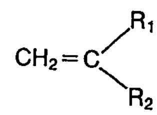

eingeleitet und, nachdem die Luft aus dem Kolben verdrängt ist, unter Rühren des Kolbeninhaltes auf die angegebene Temperatur erwärmt und für die aus der Tabelle ersichtliche Zeit auf dieser Temperatur gehalten. Dann wird die Gaszufuhr unterbrochen, der Kolben abgekühlt und von einer Probe der Reaktionsmischung durch Messung des $^{19}F$-Kernresonanzspektrums der Umsatz bestimmt. Eine weitere Probe wird gaschromatographisch auf ihre Zusammensetzung untersucht.

In der Tabelle bedeuten:

I = Gemisch verschiedener Perfluoralkyliodide mit folgender Kettenlängen-Verteilung: 37,5 Gew.-% $C_6$; 37,9 Gew.-% $C_8$; 17,5 Gew.-% $C_{10}$; 6,3 Gew.-% $C_{12}$;
II = 1-Iodperfluoroctan
VII = 1,2-Di-bromperfluorethan
a = Ethylen
$K_2$ = $[(C_2H_5O)_3P]_4Ni$
$K_3$ = $[(C_6H_5)_3P]_3RhCl$
$K_4$ = $[(C_6H_5)_3P]Cr(CO)_5$
$K_5$ = $[(C_6H_5)_3P]Mo(CO)_5$
$K_7$ = $[(C_6H_5)_3P]_4RuCl_2$
AIBN = Azo-bis-(isobutyronitril)

**Patentansprüche**

1. Verfahren zur Herstellung von fluoralkylsubstituierten Alkanen oder Alkenen durch Reaktion einer Verbindung der Formel

$$XR_fZ,$$

worin bedeuten:

$R_f$ einen perfluorierten Alkylenrest, geradkettig mit 1 bis 15 C-Atomen, verzweigt mit 3 bis 15 C-Atomen oder cyclisch mit 4 bis 8 C-Atomen,

X Wasserstoff, Fluor, Chlor, Brom oder Iod,

Z Brom oder Iod,

mit einer Verbindung der Formeln

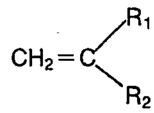

worin $R_1$ und $R_2$ gleich oder verschieden sein können und jedes für sich bedeutet: Wasserstoff, Fluor, Chlor, einen Perfluoralkylrest mit 1 bis 12 C-Atomen, in dem ein Fluoratom durch ein Wasserstoff- oder Chloratom ersetzt sein kann, einen Alkylrest mit 1 bis 20 C-Atomen, einen Alkenylrest mit 2 bis 20 C-Atomen, einen Arylrest mit 6 bis 10 C-Atomen oder einen Arylalkylrest mit 7 bis 12 C-Atomen, wobei ein Substituent der vier zuletzt genannten Arten seinerseits substituiert sein kann mit F; Cl; –OH oder –OR′, worin R′ einen Alkyl- oder Alkylcarboxy-Rest mit 1 bis 5 C-Atomen bedeutet, oder

$$CH \equiv C–R_3,$$

worin $R_3$ bedeutet: einen Perfluoralkylrest mit 1 bis 12 C-Atomen, in dem ein Fluoratom durch ein Wasserstoff- oder Chloratom ersetzt sein kann, einen Alkylrest mit 1 bis 20 C-Atomen, einen Alkenylrest mit 2 bis 20 C-Atomen, einen Arylrest mit 6 bis 10 C-Atomen oder einen Arylalkylrest mit 7 bis 12 C-Atomen, wobei ein Substituent der vier zuletzt genannten Arten seinerseits substituiert sein kann mit F; Cl; –OH oder –OR′, worin R′ einen Alkyl- oder Alkylcarboxy-Rest mit 1 bis 5 C-Atomen bedeutet, in der Wärme bei Normaldruck oder Überdruck in Gegenwart eines Metall-Komplex-Katalysators, dadurch gekennzeichnet, dass bei 20 bis 130 °C gearbeitet wird und als Katalysator mindestens eine Metall-Komplexverbindung eingesetzt wird, die als Zentralatom ein Metall enthält, welches im Periodensystem der Elemente eine der Ordnungszahlen 24 bis 30; 42 bis 48 oder 74 bis 79 hat, und eine Wertigkeitsstufe von Null, +1 oder +2 aufweist, wobei diese Metall-Komplexverbindung mindestens einen der folgenden Liganden enthält: Kohlenmonoxid; Trialkylphosphit; Triarylphosphit oder eine Verbindung der Formel

$$(R)_3Q,$$

worin bedeuten:

R = Alkyl-, Aryl- oder Arylalkyl-

Q = Phosphor, Arsen oder Antimon, wobei ferner solche Metall-Komplexverbindungen ausgeschlossen sind, die Kohlenmonoxid als Liganden und ein Metall, das im Periodensystem der Elemente eine der Ordnungszahlen 26 bis 28; 44 bis 46 und 76 bis 78 aufweist, als Zentralatom enthalten.

2. Verfahren nach Anspruch 1, dadurch gekennzeichnet, dass das als Zentralatom in der Komplexverbindung vorliegende Metall die Wertigkeitsstufen Null oder +1 aufweist.

3. Verfahren nach Anspruch 1 oder 2, wobei eine der beiden reagierenden Verbindungen, entweder das fluorierte Alkyliodid oder Alkylbromid oder das substituierte oder unsubstituierte Alken oder Alkin, in geringerer Molzahl eingesetzt wird als die andere reagierende Verbindung, dadurch gekennzeichnet, dass 0,1 bis 10 Mol-%, bezogen auf jene der beiden reagierenden Verbindungen, die mit geringerer Molzahl eingesetzt wird, von der als Katalysator verwendeten Metall-Komplexverbindung angewendet werden.

**Claims**

1. A process for preparing fluoroalkyl-substituted alkanes or alkenes by reacting a compound of the following formula

$$XR_fZ$$

in which

$R_f$ denotes a perfluorinated alkylene radical, which is straight-chain and has 1 to 15 carbon atoms or is branched and has 3 to 15 carbon atoms or is cyclic and has 4 to 8 carbon atoms,

X denotes hydrogen, fluorine, chlorine, bromine or iodine, and

Z denotes bromine or iodine,

with a compound of the following formulas

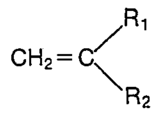

in which $R_1$ and $R_2$ can be identical or different and each denotes: hydrogen, fluorine, chlorine, a perfluoroalkyl radical which has 1 to 12 carbon atoms and in which a fluorine atom can be replaced by hydrogen or chlorine, an alkyl radical having 1 to 20 carbon atoms, an alkenyl radical having 2 to 20 carbon atoms, an aryl radical having 6 to 10 carbon atoms or an arylalkyl radical having 7 to 12 carbon atoms, and a substituent of the last mentioned four types can in turn be substituted by F, Cl, –OH or –OR′, in which R′ denotes an alkyl or alkylcarboxyl radical having 1 to 5 carbon atoms, or

$$CH \equiv C–R_3$$

in which $R_3$ denotes: a perfluoroalkyl radical of 1 to 12 carbon atoms in which a fluorine atom can be replaced by a hydrogen or chlorine atom, an alkyl radical of 1 to 20 carbon atoms, an alkenyl radical of 2 to 20 carbon atoms, an aryl radical of 6 to 10 carbon atoms or an arylalkyl radical of 7 to 12 carbon atoms and a substituent of the last mentioned four types can in turn be substituted by F, Cl, –OH or –OR′, in which R′ denotes an alkyl or alkylcarboxyl radical of 1 to 5 carbon atoms, under heat and under atmospheric pressure or superatmospheric pressure in the presence of a metal complex catalyst, characterised by carrying out the process at 20 to 130 °C and using as said catalyst at least one metal complex compound which contains as the central atom a metal which, in the periodic table of the elements, has one of the atomic numbers 24 to 30, 42 to 48 or 74 to 79 and has an oxidation state of zero, +1 or +2, and contains at least one of the following ligands: carbon monoxide, trialkyl phosphite, triaryl phosphite or a compound of the formula $(R)_3Q$ in which R denotes an alkyl, aryl or arylalkyl radical and Q denotes phosphorus, arsenic or antimony, the metal complex compounds which contain carbon monoxide as ligand and a metal which, in the periodic table of elements, has one of the atomic numbers 26 to 28, 44 to 46, and 76 to 78 are disclaimed.

2. The process as claimed in claim 1, characterised in that the metal which is present as the central atom in the complex compound is in oxidation state zero or +1.

3. The process as claimed in claim 1 or 2 in which one of the two reacting compounds, either the fluorinated alkyl iodide or alkyl bromide or the substituted or unsubstituted alkene or alkyne, is used in a smaller number of moles than the other reacting compound, characterised in that the metal complex compound used as the catalyst is used in an amount of 0.1 to 10 mole%, based in whichever of the two reacting compounds is used in the smaller number of moles.

**Revendications**

1. Procédé de préparation d'alcanes ou d'alcènes à substituants fluoroalkyliques par réaction d'un composé de formule

$$XR_fZ$$

dans laquelle

$R_f$ désigne un radical alkylène perfluoré à chaîne linéaire en $C_1$ à $C_{15}$, ramifiée en $C_3$ à $C_{15}$ ou cyclique en $C_4$ à $C_8$,

X désigne l'hydrogène, le fluor, le chlore, le brome ou l'iode et

Z le brome ou l'iode,

avec un composé de formule

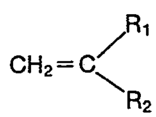

dans laquelle $R_1$ et $R_2$, qui peuvent être identiques ou différents l'un de l'autre, désignent chacun l'hydrogène, le fluor, le chlore, un radical perfluoroalkyle en $C_1$ à $C_{12}$ dont un atome de fluor peut être remplacé par un atome d'hydrogène ou de chlore, un alkyle en $C_1$ à $C_{20}$, un alcényle en $C_2$ à $C_{20}$, un aryle en $C_6$ à $C_{10}$ ou un arylalkyle en $C_7$ à $C_{12}$, l'un de ces quatre derniers substituants pouvant lui-même porter du fluor ou du chlore ou un groupe –OH ou –OR′, R′ étant un radical alkyle ou alkylcarboxy en $C_1$ à $C_5$, ou avec un composé de formule

$$CH \equiv CR_3$$

(dans laquelle $R_3$ désigne un radical perfluoroalkyle en $C_1$ à $C_{12}$ dont un atome de fluor peut être remplacé par un atome d'hydrogène ou de chlore, un alkyle en $C_1$ à $C_{20}$, un alcényle en $C_2$ à $C_{20}$, un aryle en $C_6$ à $C_{10}$ ou un arylalkyle en $C_7$ à $C_{12}$, l'un de ces quatre derniers substituants pouvant aussi porter lui-même du fluor ou du chlore ou un groupe –OH ou –OR′, R′ étant un radical alkyle ou alkylcarboxy en $C_1$ à $C_5$), à chaud et à la pression normale ou sous-pression en présence d'un complexe de métal comme catalyseur, procédé caractérisé en ce que l'on opère à des températures de 20 à 130 °C avec comme catalyseur un ou plusieurs composés complexes de métaux dont l'atome central est un métal de numéro atomique 24 à 30; 42 à 48 ou 74 à 79 et de valence 0, 1 ou 2, complexes de métaux qui comprennent un ou plusieurs des coordinats suivants:
monoxyde de carbone, phosphite de trialkyle, phosphite de triaryle ou un composé de formule:

$$(R)_3Q,$$

R désignant un alkyle, un aryle ou un aralkyle et

Q le phosphore, l'arsenic ou l'antimoine, à l'exclusion des complexes qui ont comme coordinat du monoxyde de carbone et comme atome central un métal de numéro atomique 26 à 28, 44 à 46 ou 76 à 78.

2. Procédé selon la revendication 1 caractérisé en ce que le métal constituant l'atome central du composé complexe a une valence nulle ou la valence 1.

3. Procédé selon la revendication 1 ou 2 dans lequel l'un des deux réactifs, c'est-à-dire soit le bromure ou l'iodure d'alkyle fluoré, soit l'alcène ou l'alcyne substitué ou non, est mis en réaction dans une plus faible proportion molaire que l'autre, procédé caractérisé en ce que l'on utilise de 0,1 à 10 Mol-% du composé complexe de métal servant de catalyseur par rapport à celui des deux réactifs qui est employé en plus faible proportion molaire par rapport à l'autre.